# EUROPEAN PATENT APPLICATION

(11) **EP 0 949 336 A1**
(43) Date of publication of application: **13.10.1999**
(21) Application number: 98302135.3
(22) Date of filing: 20.03.1998
(51) Int. Cl.: C12Q 1/60, G01N 33/92, C12Q 1/00, G01N 33/53

(54) **Process for identification of organic material**

(71) Applicant: UNILEVER PLC, London EC4P 4BQ (GB)
(72) Inventor: Dittmer, Monika, Lever Faberge Deutschland GmbH, 21614 Buxtehude (DE); Kruse, Jan, Lever Faberge Deutschland GmbH, 21614 Buxtehude (DE); Meyer, Frank, Lever Faberge Deutschland GmbH, 21614 Buxtehude (DE)
(74) Representative: Elliott, Peter William

(57) **Abstract**

A process is provided for identifying presence of organic material in a surfactant-containing medium using a biological assay method. Reagents of the biological assay may include enzymes or immunological agents.

## Description

The present invention relates to the use of a biological assay for identifying organic material. In particular, the invention relates to such use for identifying presence of lipids, especially lipids that are naturally found in skin.

The surface layer of skin, the stratum corneum, contains specific lipids that are produced by the skin itself to prevent surface evaporation of water and thus prevent drying out. Cosmetic compositions, such as skin creams are conventionally used to replenish these lipids in dry skin so as to maintain a healthy skin.

Recently surfactant-containing skin cleansers have been formulated to also comprise lipids naturally found in the skin (see, for example, EP-B-0 786 903) so as to maintain the level of the skin's lipids content, even after the removal of dirt and sebum from the skin. However it appears to be difficult to test for the presence of such compounds in skin cleansers.

Although enzymatic and other biological assays are known which are able to identify a large variety of components, including lipids, in serum and other body fluids, such assays were considered to be unsuitable for testing the presence of such compounds in surfactant containing media because the denaturing action of surfactants would either completely destroy the enzymes involved or so seriously reduce their catalytic activity that an effective assay would be impossible. It was also thought that other proteins such as antibodies, which can also be used to identify organic material, would also be ineffective in the presence of surfactant.

Examples of known conventional, and relatively inexpensive, tests for identifying cholesterol in serum include the liquid test kit available from Merck Inc. as the Merkoquant™ cholesterol test and that available in a strip format from Boehringer GmbH known as the AccuTrend™ test.

The reagents used in these tests are the hydrolysing and oxidising enzymes cholesterin-esterase and -oxidase respectively, which together catalyse the cascade release of hydrogen peroxide from cholesterol. The presence of hydrogen peroxide can be identified by well known colour generating reactions, such as by reaction with 4-amino-antipyrine and phenols to produce coloured chinone imines.

A wide variety of colours may be generated, for example the test mentioned above from Merck provides a positive red signal, whereas that from Bieiersdorf provides a positive blue signal.

However, there is also a need for a quick and easy way of testing the presence of natural skin-lipids in surfactant-containing media.

It has now been found that sensitive biological assays can be used for accurately identifying the presence of skin-lipids and other organic compounds in surfactant-containing media, such as cosmetic cleansers.

Thus, the present invention provides a process for identifying presence of organic material in a surfactant-containing medium using a biological assay method.

In the context of this invention, a biological assay is defined as a procedure for the quantitative or qualitative identification of the presence of organic material by means of any biological or biochemical reagent capable of detecting such organic material.

In a preferred embodiment, the reagents of the biological assay are enzymes, but immunological reagents such as antibodies can also be used.

Assays can be performed in solution or by immobilising the reagents, for example on a solid support, preferably a porous strip (for instance made of paper), a porous polymeric sheet material or another support material coated with a porous layer. In a preferred use, the assays provide a quick, visual signal when organic material is identified, and such a signal can be the generation of a colour readily perceived qualitatively or may be presented quantitatively, for example photometrically. Preferred assays are based on a multiple reaction cascade format as exemplified below.

The surfactants contained in the medium in which the organic material is identified may be any surfactant (for example anionic, amphoteric, or nonionic surfactants) or combination of surfactants well known in the art (see International Cosmetic Handbook, publ.1997).

Typically the medium contains surfactant in liquid or dissolved form, for example as an aqueous solution of shower gel, or lather generated from such a solution. Preferably the solution or lather contains no more than 50% by wt., preferably no more than 20% by wt., more preferably no more than 10% by wt., of surfactant. Further, the solution or lather preferably contains no less than 0.1% by wt., more preferably no less than 1%, still more preferably no less than 5%, of surfactant.

Although carbohydrate and protein material, vitamins, organic sunscreens or mixtures of such organic material may be identified by selecting a suitable assay which is sensitive to one or more of these materials, particularly preferred organic materials to be tested for art lipids.

Identifiable lipids may be any lipid well known in the field of cosmetic compositions (again, see the International Cosmetic Handbook mentioned above). In particular, the lipids can be natural skin-lipids, for example sphingolipids, free sterols, free fatty acids, triglycerides, or polar lipids, or mixtures of these lipids. Of the free sterols, cholesterol or their derivatives can specifically be identified.

The invention will now be illustrated by the following non-limiting uses of enzymatic assays.

Surfactant-containing liquid cosmetic compositions (listed below) currently on the market were tested with the above mentioned Merckoquant™ and AccuTrend™ kits as follows:-

### I. The Merckoquant ™ test kit

Test solutions A (colourant I), B (colourant II) and C (enzymes) were prepared according to the procedure recommended by Merck, with a final test solution being obtained by mixing solutions A, B and 1ml of C.

The compositions to be analysed were diluted 1:1 (that is, by 50% wt. of original concentration) with de-ionized water. Subsequently, 2 ml of this diluted product and 20 ml of the above final test solution were mixed vigorously in a glass tube at room temperature. After 15 seconds a red discolouration appeared if cholesterol was present in the composition being tested; the reaction was typically complete after 10 minutes.

### II. First Test Strip

The Merckoquant™ test reagents were immobilized by dropping a few mls of the final test solution (see I. above) onto thin-layer-chromatography plates (available from Sigma-Aldrich) in the form of aluminium plates coated with silica. The so prepared test strip was immersed into an aqueous solution of the compositions (diluted by 10% wt. of original concentration) to be analyzed. when cholesterol-containing compositions were tested a reddish discolouration appeared after a few seconds (end point: 10 minutes) which remained stable over several weeks.

### III. AccuTrend™ Test Strip

A few drops of an aqueous solution of the composition (diluted by 50% wt. of original concentration) to be tested was placed on the receiving matrix in the window of the test strip. Special attention was paid to completely saturate this matrix with the diluted composition to avoid any inhomogenieity in the results of colour generated during the testing of each composition.

After 10 seconds the bluish discolouration of the test window began to appear, with the reaction being complete after 3 minutes at which point the test window was completely discoloured bluish-green.

In the following compositions, which contain natural skin-lipids, a positive visual signal was generated quickly and clearly:-
Dove Cream Shower™
Rexona Sensitive™

In contrast, the following liquid products similarly tested did not give a positive visual signal:-

### Not containing natural skin-lipid

Nivea Cream Shower™
Monsavon Shower Milk™
Revlon Dry Skin Relief™ shower gel
Penaten Baby Wash™ and shower Cream™
CD Shower Balm™
Nivea Bath Care Shower Milk™
Imperial Leather Shower™ and Cream™
Garnier Nutralia 2in1™

### Not containing cholesterol

Sanex Sport™

## Claims

1. Process for identifying presence of organic material in a surfactant-containing medium wherein a biological assay method is used.

2. Process according to claim 1, wherein reagents of the biological assay comprise enzymes or immunological agents.

3. Process according to claim 2, wherein the enzymes are immobilised on a solid support.

4. Process according to claim 2, wherein the enzymes are free in solution.

5. Process according to any preceding claim, wherein the biological assay provides a quick, visual signal when the organic material is identified.

6. Process according to claim 5, wherein the signal provides a quantitative measure of the amount of organic material tested for.

7. Process according to any preceding claim, wherein the medium contains anionic, amphoteric, or nonionic surfactant, or mixtures of such surfactants.

8. Process according to any preceding claim, wherein the medium contains surfactant in liquid or dissolved form.

9. Process according to claim 8, wherein the liquid is a shower gel.

10. Process according to claim 8, wherein a lather from the dissolved form is tested.

11. Process according to any preceding claim, wherein the liquid contains no more than 50% by wt. of surfactant.

12. Process according to any preceding claim, wherein the liquid contains no more than 20% by wt. surfactant.

13. Process according to any preceding claim, wherein the liquid contains no more than 10% by wt. surfactant.

14. Process according to any preceding claim, wherein the liquid contains at least 0.1% by wt. surfactant.

15. Process according to any preceding claim, wherein the liquid contains at least 1% by wt. surfactant.

16. Process according to any preceding claim, wherein the liquid contains at least 5% by wt. surfactant.

17. Process according to any preceding claim, wherein the organic material is selected from lipids, carbohydrates, proteins, peptides, vitamins, organic sunscreens, or mixtures thereof.

18. Process according to claim 10, wherein the lipid comprises one or more of:
(a) sphingolipids,
(b) sterols or sterol derivatives,
(c) fatty acids or fatty acid derivatives,
(d) triglycerides, or
(e) polar lipids.

19. Process according to claim 11, wherein the sterol or sterol derivative is cholesterol or a cholesterol derivative.
